# EUROPÄISCHE PATENTSCHRIFT

(11) **EP 3 989 848 B1**
(45) Veröffentlichungstag und Bekanntmachung des Hinweises auf die Patenterteilung: **16.08.2023**
(21) Anmeldenummer: 20733920.1
(22) Anmeldetag: 15.06.2020
(51) Int. Cl.: A61B 17/3211, A61B 17/3213, A61B 90/70

(54) **MEDIZINISCHES HANDINSTRUMENT MIT AUSTAUSCHBARER KLINGE**
MEDICAL HAND-HELD INSTRUMENT WITH A REPLACEABLE BLADE
INSTRUMENT À MAIN MÉDICAL ÉQUIPÉ D'UNE LAME REMPLAÇABLE

(30) Priorität: 25.06.2019 DE 102019117076
(43) Veröffentlichungstag der Anmeldung: 04.05.2022
(73) Patentinhaber: Aesculap AG, 78532 Tuttlingen (DE)
(72) Erfinder: SCHALLER, Michelle, 78054 Villingen-Schwenningen (DE); HOFMANN, Alina, 78582 Balgheim (DE)
(74) Vertreter: Winter, Brandl - Partnerschaft mbB
(86) Internationale Anmeldenummer: PCT/EP2020/066498
(87) Internationale Veröffentlichungsnummer: WO 2020/260054

(56) Entgegenhaltungen:
- WO-A1-2004/002335
- WO-A2-2010/132027
- JP-A- 2000 185 050
- JP-A- 2007 061 429
- US-A- 4 660 287
- US-B2- 8 074 364

## Beschreibung

### Technisches Gebiet

Die vorliegende Erfindung betrifft ein Skalpell mit einem vorzugsweise einstückig ausgebildeten Griff, welcher an seinem distalen Endabschnitt ein griffseitiges Positionierelement, vorzugsweise einen Positioniervorsprung, aufweist, einem an dem Griff angeordneten, austauschbaren Werkzeug, vorzugsweise einer Klinge, welches ein werkzeugseitiges Positionierelement, vorzugsweise eine Positionierausnehmung, aufweist, in welches das griffseitige Positionierelement im Wesentlichen passgenau eingreift, und einem in einer Grifflängsrichtung relativ zu dem Griff verschiebbaren und den Griff zumindest teilweise umgreifenden Sicherungsschieber, wobei der Sicherungsschieber das Werkzeug in einer Fixierposition an den Griff unmittelbar sowie federelastisch anlegt und dieses auf dem Griff fixiert und in einer Reinigungsposition freigibt, dadurch gekennzeichnet, dass der Griff im Bereich der Reinigungsposition eine gegenüber dem distalen Endabschnitt reduzierte Griffstärke aufweist.

### Stand der Technik

Aus dem Stand der Technik sind bereits Skalpelle dieser Gattung bekannt, bei welchen zur Montage bzw. Demontage der Klinge an dem Griff eine Handkraft auf die Klinge aufgebracht werden muss. So zeigt die GB 265 425 A beispielsweise ein Skalpell mit einem Skalpellgriff, der an einem distalen Endabschnitt seitlich zwei Führungsschienen zur Aufnahme einer Skalpellklinge aufweist. Die Skalpellklinge wird dabei zur Montage und Demontage aufgeschoben bzw. abgezogen.

Des Weiteren offenbart die US 2 708 313 A ein Skalpell, bei dem die Skalpellklinge auf dem Griff durch eine rotierende Bewegung fixiert bzw. gelöst wird.

Ein weiteres Beispiel eines Skalpells mit durch Ausübung einer Handkraft auf die Klinge montier- bzw. demontierbarer Skalpellklinge ist in der DE 10 2009 007 292 A1 offenbart. Insbesondere weisen die Skalpellklinge und der Skalpellgriff dabei jeweils Kupplungselemente auf, die durch kraft- und/oder formschlüssigen Eingriff miteinander die Skalpellklinge auf dem Skalpellgriff fixieren.

Bei allen vorstehend genannten Skalpellen muss jedoch zur Montage bzw. Demontage der Skalpellklinge auf diese eine Handkraft ausgeübt werden. Dabei besteht die Gefahr, dass ein Anwender sowohl beim Fügen als auch beim Entfernen der Skalpellklinge abrutschen und sich dabei verletzen kann. Vor allem beim Entfernen der verschmutzten bzw. kontaminierten Skalpellklinge nach operativem Gebrauch steigt dadurch das Infektionsrisiko für den Anwender.

Daher sind aus dem Stand der Technik Skalpelle bekannt, welche die Montage bzw. Demontage der Skalpellklinge ohne Ausübung einer Handkraft auf diese ermöglichen. Insbesondere die US 2017 112 522 A offenbart ein Skalpell, das die Fixierung der Skalpellklinge durch Rotation des Skalpellgriffs relativ zu der Skalpellklinge ermöglicht. Jedoch können sich in dem hier zwischen der Skalpellklinge und dem Skalpellgriff ausgebildeten Schwenkgelenk Verunreinigungen sammeln, was die Reinigung des Skalpells erschwert.

Auch zeigt die EP 2 752 275 A ein weiteres Skalpell mit möglicher Montage und Demontage der Skalpellklinge, ohne Ausübung einer Handkraft. Dabei wird zunächst die Skalpellklinge auf den Skalpellgriff aufgelegt, so dass ein feststehender und ein verschiebbarer Bolzen am Griff in eine langlochförmige Aussparung in der Skalpellklinge greift. Die Aussparung ist dabei mit im Wesentlichen zwei unterschiedlichen Breiten ausgestaltet, von denen die größere Breite im Wesentlichen der Breite der Bolzen entspricht. Durch Verschieben des verschiebbaren Bolzens wird die Skalpellklinge relativ zu dem feststehenden Bolzen bewegt und die Skalpellklinge gleitet in an dem feststehenden Bolzen seitlich ausgebildeten Führungsnuten, was die Fixierung der Skalpellklinge ermöglicht. Nachteilig ist hierbei jedoch, dass die Skalpellklinge zusätzlich verspannt wird und die Ausgestaltung der Verschiebemechanik als im Griff angeordneter Schlitten die Reinigung des Skalpells erschwert. Zudem zeigen WO 2010 / 132 027 A2, US 4 660 287 A, JP 2000 - 180 050 A oder WO 2004 / 002 335 A1 Beispiele für Skalpelle mit axial verlagerbaren Sicherungsschiebern gemäß dem Oberbegriff des Anspruchs 1.

Ein weiteres Beispiel für ein Skalpell, bei welchem die Montage bzw. Demontage der Skalpellklinge ohne Ausübung einer Handkraft auf diese ermöglicht, ist in der US 1 625 778 A offenbart. Dabei weist der Skalpellgriff einen Hauptkörper und einen relativ dazu verschwenkbaren Schwenkarm auf. Die Skalpellklinge wird in den Hauptkörper eingelegt und durch den Schwenkarm gehalten. Zur Fixierung des Schwenkarms und damit der Skalpellklinge in dem Skalpellgriff wird ein relativ zu dem Skalpellgriff verschiebbarer Ring an dem Skalpellgriff in Richtung der Skalpellklinge verschoben. Da der Ring den Skalpellgriff vollständig umgreift und im Wesentlichen daran anliegt, besteht bei der Reinigung des Skalpells die Gefahr, dass der Bereich zwischen dem Ring und dem Skalpell nicht oder nur schlecht gespült bzw. gereinigt werden kann.

Mit anderen Worten, muss eine Skalpellklinge, um einen verwendbaren Skalpellgriff mit der Skalpellklinge in einer Operation benutzen zu können, zuvor vom OP-Personal auf den Skalpellgriff montiert werden. Bei den vorstehend genannten Skalpellgriffen ist hierfür zumeist eine direkte Handkraft auf die Klinge erforderlich. Damit die verschmutzte Klinge (verschmutzt mit Blut und Geweberesten) nach dem Gebrauch entsorgt werden kann, muss diese wieder vom Griff entnommen werden. Sowohl das Fügen als auch das Entfernen der Klinge stellt für den Anwender ein hohes Verletzungsrisiko dar, da der Anwender bei beiden Vorgängen schnell abrutschen und sich somit schneiden kann.

### Kurzbeschreibung der Erfindung

Es ist also die Aufgabe der Erfindung, die Nachteile aus dem Stand der Technik zu vermeiden oder wenigstens zu mildern. Insbesondere soll ein medizinisches Handinstrument, insbesondere ein Skalpell, mit einer Klinge und einem Griff bereitgestellt werden, das eine Montage und Demontage der Klinge an bzw. von dem Griff ohne Ausübung einer Handkraft auf die Klinge durch den Anwender ermöglicht und gleichzeitig eine ausreichende Reinigbarkeit gewährleistet.

Diese Aufgabe wird bei einer gattungsgemäßen Vorrichtung erfindungsgemäß durch die Merkmale des Patentanspruchs 1 und insbesondere dadurch gelöst, dass der Sicherungsschieber bzw. die Sicherungshülse die Klinge in einer Fixierposition auf dem Griff durch unmittelbare Anlage an diesem fixiert und in einer Reinigungsposition, in welcher sich zwischen dem Sicherungsschieber und dem Griff vorzugsweise ein Spülspalt ergibt, freigibt.

Dies hat den Vorteil, dass die Klinge ohne Ausübung einer Handkraft auf die Klinge auf dem Griff positioniert und durch die Sicherungshülse fixiert werden kann. Gleichzeitig bleibt eine ausreichende Reinigbarkeit des Griffs bei demontierter Klinge gewährleistet.

In anderen Worten ausgedrückt, ist es erfindungsgemäß vorgesehen, den Skalpellgriff mit einem Klingenaufnahmeabschnitt auszubilden, an dem ein mit der Klinge zusammenwirkendes (einrast- oder einschnapploses) Klingen-Positionierelement angeordnet ist. Des Weiteren ist am Skalpellgriff ein vorzugsweise hülsenförmiger (oder klammerartiger) Sicherungsschieber längsverschieblich gelagert, der in einer (vorgeschobenen) Fixierposition eine (elastische) Andrückkraft auf die Klinge ausübt und diese somit im Klingenaufnahmeabschnitt festhält. Schließlich ist der Sicherungsschieber aus der Fixierposition in eine Freigabe- oder Reinigungsposition (rück-) schiebbar, in welcher die Klinge (weil einrastlos) einfach vom Klingenaufnahmeabschnitt abnehmbar ist oder sogar selbsttätig abfällt. Der Skalpellgriff weist zudem im Bereich der Freigabe- oder Reinigungsposition vorzugsweise eine gegenüber dem Klingenaufnahmeabschnitt reduzierte Griffstärke (Griffdicke) auf, wodurch sich zwischen dem Skalpellgriff und dem Sicherungsschieber ein Spül- bzw. Reinigungsspalt ergibt.

Vorteilhafte Ausführungsformen sind in den Unteransprüchen beansprucht und werden nachfolgend näher erläutert.

Vorzugsweise kann die Sicherungshülse/Sicherungsschieber zumindest eine zu dem Griff hin vorstehende Fixierungsnoppe aufweisen und der Griff zumindest eine die zumindest eine Fixierungsnoppe in der Grifflängsrichtung führende Führungslängsnut aufweisen. Dabei kann der Griff an seinem distalen Endabschnitt, d.h. im Klingenaufnahmeabschnitt, des Weiteren eine Schräge bzw. Rampe aufweisen, welche in Richtung proximal abfällt. Die zumindest eine Fixierungsnoppe kann somit die Klinge bei einer Verschiebung des Sicherungsschiebers in Richtung Fixierposition zunehmend gegen den Griff, insbesondere gegen die Schräge bzw. Rampe, drücken. Dadurch kann sichergestellt werden, dass die Klinge und die Fixierungsnoppe erst in der Fixierposition klingen-festhaltend in Kontakt treten, was eine genaue Ausrichtung der Klinge auch noch während der Schiebebewegung gewährleistet.

In einer bevorzugten Ausgestaltung kann die Sicherungshülse/Sicherungsschieber ein Federblech/Federzunge aufweisen, welches in der Fixierposition in eine auf dem Griff ausgebildete Federblechnut greift, um das Sicherungsblech gegen WeiterVerschieben in Richtung distal zu sichern. Dies hat den Vorteil, dass die Klinge relativ zu dem Griff an einer vorbestimmten Position und mit definierter Anpresskraft fixiert wird und somit eine ordnungsgemäße Ausrichtung der Klinge sichergestellt werden kann. Außerdem wird ein Abziehen des Sicherungsschiebers in Richtung distal wirksam verhindert.

Erfindungsgemäß weist, wie vorstehend bereits ausgeführt, der Griff an seinem proximalen Endabschnitt einen Verjüngungsabschnitt auf, dessen Ausdehnung in Grifflängsrichtung im Wesentlichen einer Länge der Sicherungshülse/Sicherungsschiebers entspricht, so dass zwischen der Sicherungshülse und dem Griff ein Spalt ausgebildet ist, wenn sich die Sicherungshülse in der Reinigungsposition befindet. Dadurch kann sichergestellt werden, dass eine Reinigungsflüssigkeit den Griff und die Sicherungshülse vollumfänglich umspülen kann und das Skalpell maschinell aufbereitet und ordnungsgemäß gereinigt werden kann.

In einer bevorzugten Weiterbildung kann das griffseitige Positionierelement einrast- oder einschnappfrei in das werkzeugseitige Positionierelement eingreifen.

Bei einem erfindungsgemäßen medizinischen Handinstrument kann das klingenseitige Positionierelement in Form eines Langlochs ausgestaltet sein, dessen Breite sich vorzugsweise stufenweise von einer ersten Breite auf eine zweite Breite ändert. Somit kann die Kompatibilität mit herkömmlichen Skalpellgriffen gewährleistet werden.

Gemäß einer vorteilhaften Weiterbildung kann die Sicherungshülse/Sicherungsschieber auf ihrer Außenseite eine Vielzahl an Noppen aufweisen, was die Haptik des Skalpells verbessert, indem ein Abrutschen des Anwenders verhindert und somit die Sicherheit erhöht wird.

### Kurzbeschreibung der Figuren

Die Erfindung wird nachstehend anhand eines bevorzugten Ausführungsbeispiels unter Bezugnahme auf die begleitenden Figuren näher beschrieben.
Fig. 1 ist eine perspektivische Vorderansicht eines Skalpells gemäß einem bevorzugten Ausführungsbeispiel der vorliegenden Offenbarung in einer Fixierposition;
Fig. 2 ist eine perspektivische Rückansicht des Skalpells gemäß dem bevorzugten Ausführungsbeispiel der vorliegenden Offenbarung in der Fixierposition;
Fig. 3 ist eine perspektivische Vorderansicht des Skalpells gemäß dem bevorzugten Ausführungsbeispiel der vorliegenden Offenbarung in einer Reinigungsposition;
Fig. 4 ist eine perspektivische Vorderansicht eines Skalpellgriffs gemäß dem bevorzugten Ausführungsbeispiel der vorliegenden Offenbarung;
Fig. 5 ist eine perspektivische Rückansicht des Skalpellgriffs gemäß dem bevorzugten Ausführungsbeispiel der vorliegenden Offenbarung;
Fig. 6 ist eine perspektivische Vorderansicht eines Sicherungsblechs gemäß dem bevorzugten Ausführungsbeispiel der vorliegenden Offenbarung;
Fig. 7 ist eine Teilansicht eines Klingenaufnahmebereichs des Skalpells gemäß dem bevorzugten Ausführungsbeispiel der vorliegenden Offenbarung; und
Fig. 8 ist eine seitliche Teilansicht des Klingenaufnahmebereichs des Skalpells gemäß dem bevorzugten Ausführungsbeispiel der vorliegenden Offenbarung.

### Beschreibung des bevorzugten Ausführungsbeispiels

Nachstehend wird ein bevorzugtes Ausführungsbeispiel der vorliegenden Offenbarung auf der Basis der zugehörigen Figuren beschrieben. Die Figuren sind lediglich schematischer Natur und dienen ausschließlich dem Verständnis der Erfindung. Die gleichen Elemente sind mit denselben Bezugszeichen versehen.

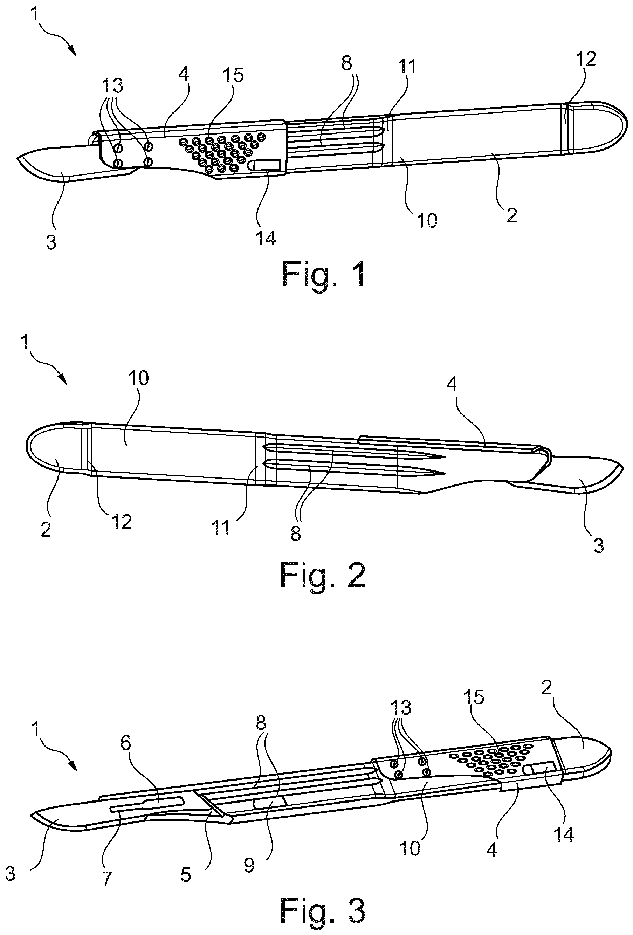

Fig. 1 und Fig. 2 sind perspektivische Vorder- und Rückansichten eines Skalpells 1 gemäß einem bevorzugten Ausführungsbeispiel der vorliegenden Offenbarung in einer Fixierposition. Das Skalpell 1 weist dabei einen Skalpellgriff 2, eine an dem Skalpellgriff 2 lösbar befestigte Skalpellklinge 3 und ein zur Fixierung der Skalpellklinge 3 verwendetes Sicherungsblech (Sicherungsschieber) 4 auf.

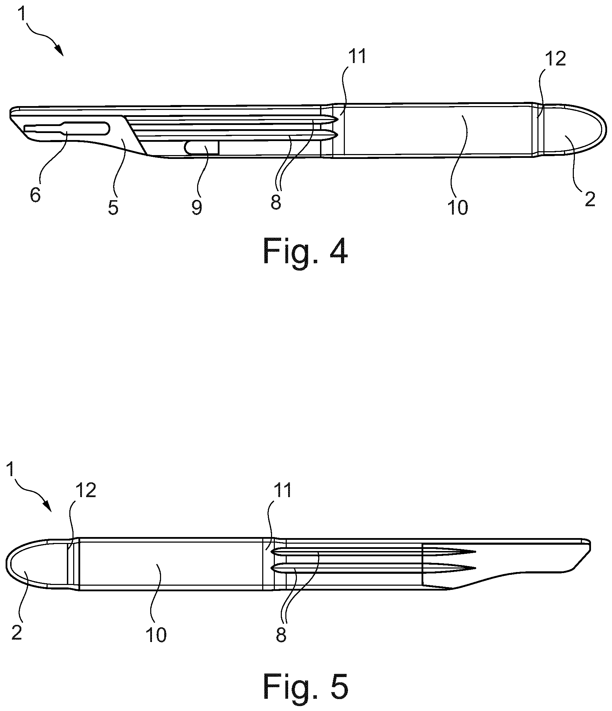

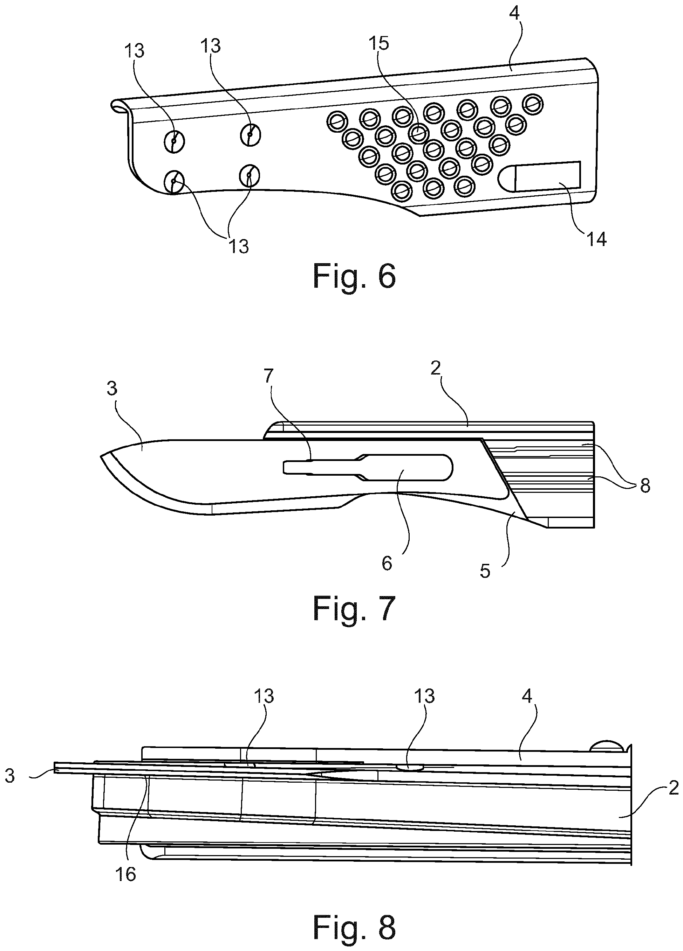

Wie in Fig. 4 und Fig. 5 zu erkennen, ist der Skalpellgriff 2 im Wesentlichen als ein langgestreckter, flacher quaderförmiger Körper ausgestaltet, an dessen distalem Endabschnitt (klingenseitiges Ende) ein sich zu dem distalen Endabschnitt hin verjüngender Klingenaufnahmeabschnitt 5 zur Aufnahme der Skalpellklinge 3 ausgebildet ist. An dem Klingenaufnahmeabschnitt 5 ragt an einer Flachseite des Griffs 2 ein Positioniervorsprung (Nocke) 6 von der Skalpellgriffoberfläche nach außen vor. Dieser Positioniervorsprung 6 ist in dem bevorzugten Ausführungsbeispiel so ausgestaltet, dass er im Wesentlichen passgenau, vorzugsweise ohne Hinterschnitt in Einlegerichtung, in eine in der Skalpellklinge 3 ausgebildete Positionieraussparung/Positioniernut 7 greift, so dass die Skalpellklinge 3 durch Eindringen des Positioniervorsprungs 6 in die Positionieraussparung 7, ohne Ausübung einer Handkraft durch einen Anwender auf die Skalpellklinge 3, relativ zu dem Skalpellgriff 2 ausgerichtet werden kann. Wie insbesondere in Fig. 7 zu erkennen, ist die Positionieraussparung 7 langlochförmig ausgebildet, wobei sich die Breite der langlochförmigen Positionieraussparung 7 zur Spitze der Skalpellklinge 3 hin stufenweise von einer ersten Breite auf eine zweite Breite verringert. Dementsprechend ist der Positioniervorsprung 6 geformt, um eine möglichst spielfreie Passung mit der Positionieraussparung 7 einzugehen.

In einem Mittenabschnitt des Skalpellgriffs 2 sind in dem bevorzugten Ausführungsbeispiel auf beiden Flachseiten des Skalpellgriffs 2 jeweils zwei sich in Skalpellgrifflängsrichtung erstreckende Führungsnuten 8 ausgebildet. Darüber hinaus weist der Skalpellgriff 2 im Bereich der Führungsnuten 8 eine rampenförmige Federblechnut 9 auf, welche einen Axialanschlag definiert. Von dem Mittenabschnitt hin zu dem proximalen Endabschnitt des Skalpellgriffs 2 hat der Skalpellgriff 2 einen Verjüngungsabschnitt 10 mit verringerter Griffstärke, der an seinem distalen Ende durch einen ersten Absatz 11 und an seinem proximalen Ende durch einen zweiten Absatz 12 von der regulären Griffstärke des Skalpellgriffs 2, vorzugsweise beidseitig, abgesetzt ist.

In Fig. 6 ist perspektivisch das Sicherungsblech 4 des Skalpells gemäß dem bevorzugten Ausführungsbeispiel abgebildet. Das Sicherungsblech 4 ist aus einer flachen Platte ausgebildet, deren lange Seiten/Längskanten (um 180°) umgebogen sind, so dass diese den Skalpellgriff 2 im Bereich seines Mittenabschnitts im Wesentlichen gleitfähig und nutenartig umgreifen. Somit ist das Sicherungsblech 4 in der Skalpellgrifflängsrichtung relativ zu dem Skalpellgriff 2 verschiebbar. Mit anderen Worten, dient der Skalpellgriff 2 im Bereich seines Mittenabschnitts als Führung beim Verschieben des Sicherungsblechs 4.

An einem distalen Endabschnitt weist das Sicherungsblech 4 in dem bevorzugten Ausführungsbeispiel vier auf eine Innenseite des Sicherungsblechs 4 gerichtete Führungsnoppen 13 auf, die in den Ecken eines imaginären Rechtecks positioniert sind. Außerdem ist, wie in Fig. 6 zu erkennen, an dem Sicherungsblech 4 in dessen proximalen Endabschnitt ein von der Innenseite des Sicherungsblechs 4 wegragendes Federblech (Federzunge) 14 und mittig eine Vielzahl an auf eine Außenseite des Sicherungsblechs 4 vorragenden Noppen 15 ausgebildet. Die Noppen 15 verbessern dabei beim Verschieben des Sicherungsblechs 4 relativ zu dem Skalpellgriff 2 die Haptik und Handhabbarkeit des Skalpells 1 und verringern so die Gefahr des Abrutschens und das Verletzungsrisiko.

In dem bevorzugten Ausführungsbeispiel ist das Sicherungsblech 4 so ausgestaltet, dass lediglich eine der beiden langen Seiten/Längskanten, wie vorstehend genannt, über die gesamte Sicherungsblechlänge unter Ausbildung einer inneren Gleitnut umgebogen ist. Auf der anderen Seite/Längskante ist das Sicherungsblech 4 lediglich in einem proximalen Endabschnitt umgebogen, jedoch von dem Bereich der Noppen 15 bis hin zu dem distalen Endabschnitt des Sicherungsblechs 4 bogenförmig ausgeschnitten. Wie in Fig. 1 zu erkennen, folgt die Form des distalen Endabschnitts des Sicherungsblechs 4 daher in der Fixierposition im Wesentlichen der Form des Klingenaufnahmeabschnitts 5, so dass der Blick auf die Schneide der Skalpellklinge 3 zu keinem Zeitpunkt durch das Sicherungsblech 4 verdeckt ist.

Wenn die Skalpellklinge 3 nun in den Skalpellgriff 2 eingelegt werden soll, muss das Sicherungsblech 4 zunächst in Richtung hin zum proximalen Endabschnitt des Skalpellgriffs 2 verschoben werden, so dass der Klingenaufnahmeabschnitt 5, wie in Fig. 3 gezeigt, frei zugänglich ist. Daraufhin kann die Skalpellklinge 3 ohne Ausübung einer Handkraft durch den Anwender auf den Klingenaufnahmeabschnitt 5 seitlich aufgelegt werden. Um die Skalpellklinge 3 ordnungsgemäß auf dem Skalpellgriff 2 zu positionieren, wird die Skalpellklinge 3 so ausgerichtet, dass der Positioniervorsprung 6 des Skalpelgriffs 2 in die Positionieraussparung 7 der Skalpellklinge 3 greift/eindringt.

Zur Sicherung und Fixierung der Skalpellklinge 3 auf dem Skalpellgriff 2, wird daraufhin das Sicherungsblech 4 entlang des Skalpellgriffs 2 in die Fixierposition in Richtung distal verschoben. Dabei wird das Sicherungsblech 4 durch die in den Führungsnuten 8 des Griffs 3 laufenden/gleitenden Führungsnoppen 13 geführt, bis schließlich das von der Innenseite des Sicherungsblechs 4 wegragende Federblech 14 in der Federblechnut 9 des Griffs einrastet. Die Federblechnut 9 bildet also einen axialen Anschlag für das Federblech 14 in Richtung distal.

In dem bevorzugten Ausführungsbeispiel ist der Skalpellgriff 2, wie in Fig. 9 gezeigt, im Bereich des Klingenaufnahmeabschnitts 5 mit einer Schräge oder Rampe 16 versehen. Die Skalpellgriffoberfläche steigt dabei zum distalen Endabschnitt des Skalpellgriffs 2 hin an, so dass die Führungsnoppen 13 im Wesentlichen erst in der Fixierposition mit der Skalpellklinge 3 in einen diese endgültig festhaltenden Kontakt treten, wodurch ein ungewolltes Verschieben/Verrutschen der Skalpellklinge 3 durch die Führungsnoppen 13 verhindert werden kann.

Um die Skalpellklinge 3 nun nach operativem Gebrauch wieder von dem Skalpellgriff 2 zu lösen, wird zunächst das eingerastete Federblech 14 wieder gelöst und daraufhin das Sicherungsblech 4 in die Reinigungsposition verschoben. Die Skalpellklinge 3 kann im Anschluss daran ohne Ausübung der Handkraft auf die Skalpellklinge 3 durch den Anwender entfernt werden. Alternativ kann das Skalpell 1 gedreht werden, so dass die Skalpellklinge 3 nach Lösen der Fixierung durch das Verschieben des Sicherungsblechs 4 von dem Skalpellgriff 2 selbsttätig abfällt.

Wenn sich das Sicherungsblech 4 in der Reinigungsposition (am Verjüngungsabschnitt 10) befindet, entsteht aufgrund der reduzierten Querschnittsfläche/Griffstärke des Verjüngungsabschnitts 10 zwischen dem Sicherungsblech 4 und dem Skalpellgriff 2 ein Spalt, der einer Reinigungsflüssigkeit erlaubt, in den Zwischenraum zwischen Skalpelgriff 2 und Sicherungsblech 4 zu strömen. Dadurch kann die Reinigbarkeit des Skalpells 1 erhöht werden.

In anderen Worten ausgedrückt, wird die Skalpellklinge 3 zur Positionierung auf den Skalpellgriff 2 lediglich gelegt und nicht längsverschoben (erhabener Absatz/Positioniervorsprung 6 passend zur Positionieraussparung/Klingennut 7). Zur Fixierung der Skalpellklinge 3 wird indessen die Sicherungshülse 4 nach vorne geschoben. Sobald die Endposition/Fixierposition der Sicherungshülse 4 erreicht ist, rastet das Federblech 12 in den Skalpellgriff 2 ein. Die vier eingedrückten Führungsnoppen 13 auf der Innenseite der Sicherungshülse 4 und die leichte Schräge/Rampe 16 im Skalpellgriff 2 klemmen die Skalpellklinge 3 während dem Vorschieben der Sicherungshülse 4 federelastisch ein. Dadurch wird gewährleistet, dass sich während des Gebrauchs der Skalpellklinge 3 nicht vom Skalpellgriff 2 lösen kann. Nach der Anwendung kann das Federblech 14 gelöst und die Sicherungshülse 4 wieder nach hinten in Richtung proximal geschoben werden. Die benutzte Skalpellklinge 3 kann einfach entnommen oder ausgeworfen werden.

## Patentansprüche

1. Skalpell (1) mit einem vorzugsweise einstückig ausgebildeten Griff (2), welcher an seinem distalen Endabschnitt ein griffseitiges Positionierelement (6), vorzugsweise einen Positioniervorsprung, aufweist, einem an dem Griff (2) angeordneten, austauschbaren Werkzeug, vorzugsweise einer Klinge (3), welches ein werkzeugseitiges Positionierelement (7), vorzugsweise eine Positionierausnehmung, aufweist, in welches das griffseitige Positionierelement (6) im Wesentlichen passgenau eingreift, und einem in einer Grifflängsrichtung relativ zu dem Griff (2) zwischen einer Fixierposition und einer Reinigungsposition verschiebbaren und den Griff (2) zumindest teilweise umgreifenden Sicherungsschieber (4), wobei der Sicherungsschieber (4) das Werkzeug (3) in der Fixierposition an den Griff (2) unmittelbar sowie federelastisch anlegt und dieses auf dem Griff (2) fixiert und in der Reinigungsposition freigibt, **dadurch gekennzeichnet, dass** der Griff (2) im Bereich der Reinigungsposition des Sicherungsschiebers (4) eine gegenüber dem distalen Endabschnitt reduzierte Griffstärke aufweist, wodurch sich zwischen dem Griff (2) und dem Sicherungsschieber (4) ein Spül- bzw. Reinigungsspalt ergibt.

2. Skalpell (1) nach Anspruch 1, **dadurch gekennzeichnet, dass** der Sicherungsschieber (4) zumindest eine zu dem Griff (2) hin vorstehende Fixierungsnoppe (13) aufweist und der Griff (2) zumindest eine die zumindest eine Fixierungsnoppe (13) in der Grifflängsrichtung führende Führungsnut (8) aufweist.

3. Skalpell (1) nach Anspruch 2, **dadurch gekennzeichnet, dass** der Griff (2) an seinem distalen Endabschnitt eine Schräge oder Rampe (16) aufweist und die zumindest eine Fixierungsnoppe (13) das Werkzeug (3) in der Fixierposition gegen den Griff (2), insbesondere gegen die Schräge oder Rampe (16), drückt.

4. Skalpell (1) nach einem der Ansprüche 1 bis 3, **dadurch gekennzeichnet, dass** der Sicherungsschieber (4) ein Federblech oder Federzunge (14) hat, welches in der Fixierposition in eine an dem Griff (2) als axialer Anschlag ausgebildete Federblechnut (9) greift.

5. Skalpell (1) nach einem der Ansprüche 1 bis 4, **dadurch gekennzeichnet, dass** der Griff (2) an seinem proximalen Endabschnitt einen Verjüngungsabschnitt (10) aufweist, dessen Ausdehnung in Grifflängsrichtung im Wesentlichen einer Länge des Sicherungsschiebers (4) entspricht.

6. Skalpell (1) nach einem der Ansprüche 1 bis 5, **dadurch gekennzeichnet, dass** das griffseitige Positionierelement (6) in Einlegrichtung des Werkzeugs (3) gesehen hinterschnittfrei in das werkzeugseitige Positionierelement (7) greift.

7. Skalpell (1) nach einem der Ansprüche 1 bis 6, **dadurch gekennzeichnet, dass** das werkzeugseitige Positionierelement (7) in Form eines Langlochs ausgestaltet ist.

8. Skalpell (1) nach einem der Ansprüche 1 bis 7, **dadurch gekennzeichnet, dass** der Sicherungsschieber (4) auf seiner Außenseite eine Vielzahl an Noppen (15) aufweist.

## Claims

1. A scalpel (1) with a handle (2), preferably formed in one piece, which has at its distal end portion a positioning element (6) on the handle side, preferably a positioning projection, an exchangeable tool arranged on the handle (2), preferably a blade (3), which has a positioning element (7) on the tool side, preferably a positioning recess, in which the positioning element (6) on the handle side engages with a substantially accurate fit, and a safety slide (4) which at least partially encompassing the handle (2) and which is displaceable in a longitudinal direction of the handle (2) relative to the handle (2) between a fixed position and a cleaning position, wherein the safety slide (4) directly and resiliently applies the tool (3) to the handle (2) in the fixed position and fixes it on the handle (2) and releases it in the cleaning position, **characterized in that** the handle (2) has a reduced handle thickness in the region of the cleaning position of the safety slide (4) compared with the distal end portion, as a result of which a flushing or cleaning gap is created between the handle (2) and the safety slide (4).

2. The scalpel (1) according to claim 1, **characterized in that** the safety slide (4) has at least one fixing knob (13) projecting towards the handle (2) and the handle (2) has at least one guiding groove (8) guiding the at least one fixing knob (13) in the longitudinal direction of the handle.

3. The scalpel (1) according to claim 2, **characterized in that** the handle (2) has a slope or ramp (16) at its distal end portion and the at least one fixing knob (13) presses the tool (3) in the fixed position against the handle (2), in particular against the slope or ramp (16).

4. The scalpel (1) according to one of claims 1 to 3, **characterized in that** the safety slide (4) has a spring plate or spring tongue (14) which, in the fixed position, engages in a spring plate groove (9) formed on the handle (2) as an axial stop.

5. The scalpel (1) according to one of claims 1 to 4, **characterized in that** the handle (2) has at its proximal end portion a taper portion (10), the extent of which in the longitudinal direction of the handle substantially corresponds to a length of the safety slide (4).

6. The scalpel (1) according to one of the claims 1 to 5, **characterized in that** the positioning element (6) on the handle side engages in the positioning element (7) on the tool side without undercut as seen in the insertion direction of the tool (3).

7. The scalpel (1) according to one of claims 1 to 6, **characterized in that** the positioning element (7) on the tool side is configured in the form of an elongated hole.

8. The scalpel (1) according to one of claims 1 to 7, **characterized in that** the safety slide (4) has a plurality of knobs (15) on its outer side.

## Revendications

1. Scalpel (1) comportant un manche (2) réalisé de préférence d'un seul tenant et présentant, au niveau de sa section terminale distale, un élément de positionnement (6) côté manche, de préférence une saillie de positionnement, un outil remplaçable disposé sur le manche (2), de préférence une lame (3), qui présente un élément de positionnement (7) côté outil, de préférence un évidement de positionnement, dans lequel l'élément de positionnement (6) côté manche vient en prise de manière sensiblement parfaite, et un coulisseau de sécurité (4) pouvant se déplacer dans une direction longitudinale de manche par rapport au manche (2) entre une position de fixation et une position de nettoyage et entourant au moins partiellement le manche (2), le coulisseau de sécurité (4) plaçant l'outil (3) dans la position de fixation sur le manche (2) directement et de manière élastique et fixant celui-ci sur le manche (2) et le libérant dans la position de nettoyage,
**caractérisé en ce que** le manche (2) présente, dans la zone de la position de nettoyage du coulisseau de sécurité (4), une épaisseur de manche réduite par rapport à la section terminale distale, moyennant quoi une fente de rinçage ou de nettoyage est créée entre le manche (2) et le coulisseau de sécurité (4).

2. Scalpel (1) selon la revendication 1, **caractérisé en ce que** le coulisseau de sécurité (4) présente au moins un ergot de fixation (13) faisant saillie vers le manche (2) et le manche (2) présente au moins une rainure de guidage (8) guidant l'au moins un ergot de fixation (13) dans la direction longitudinale de manche.

3. Scalpel (1) selon la revendication 2, **caractérisé en ce que** le manche (2) présente un biseau ou une rampe (16) au niveau de sa section terminale distale et l'au moins un ergot de fixation (13) presse l'outil (3) contre le manche (2), en particulier contre le biseau ou la rampe (16), dans la position de fixation.

4. Scalpel (1) selon l'une des revendications 1 à 3, **caractérisé en ce que** le coulisseau de sécurité (4) comprend une tôle élastique ou une languette élastique (14) qui, dans la position de fixation, entre en prise dans une rainure de tôle élastique (9) réalisée au niveau du manche (2) sous la forme d'une butée axiale.

5. Scalpel (1) selon l'une des revendications 1 à 4, **caractérisé en ce que** le manche (2) présente, au niveau de sa section terminale proximale, une section effilée (10) dont l'extension dans la direction longitudinale de manche correspond sensiblement à une longueur du coulisseau de sécurité (4).

6. Scalpel (1) selon l'une des revendications 1 à 5, **caractérisé en ce que** l'élément de positionnement (6) côté manche, vu dans le sens d'insertion de l'outil (3), entre en prise sans contre-dépouille dans l'élément de positionnement (7) côté outil.

7. Scalpel (1) selon l'une des revendications 1 à 6, **caractérisé en ce que** l'élément de positionnement (7) côté outil est conçu sous la forme d'un trou oblong.

8. Scalpel (1) selon l'une des revendications 1 à 7, **caractérisé en ce que** le coulisseau de sécurité (4) présente une pluralité d'ergots (15) sur sa face extérieure.
